## Europäisches Patentamt
## European Patent Office
## Office européen des brevets

(19)

(11) Publication number : **0 351 411 B1**

# (12) EUROPEAN PATENT SPECIFICATION

(45) Date of publication of patent specification :
**04.03.92 Bulletin 92/10**

(51) Int. Cl.⁵ : **A61F 2/04**

(21) Application number : **88902597.9**

(22) Date of filing : **08.03.88**

(86) International application number :
**PCT/SE88/00108**

(87) International publication number :
**WO 88/06871 22.09.88 Gazette 88/21**

(54) **NERVE SPLICING PROSTHESIS.**

(30) Priority : **09.03.87 SE 8700968**

(43) Date of publication of application :
**24.01.90 Bulletin 90/04**

(45) Publication of the grant of the patent :
**04.03.92 Bulletin 92/10**

(84) Designated Contracting States :
**AT BE CH DE FR GB IT LI LU NL SE**

(56) References cited :
**WO-A-84/03035**
**WO-A-85/00511**

(73) Proprietor : **Astra Meditec AB**
**Box 14**
**S-431 21 Mölndal (SE)**

(72) Inventor : **BOWALD, Staffan, Folke**
**Ärentunavägen 18**
**S-743 00 Storvreta (SE)**

(74) Representative : **Kummelsten, Per-Arne**
**UPPSALA PATENTBYRA P.O. Box 9013**
**S-750 09 Uppsala (SE)**

## Description

The present invention relates to the splicing of nerves, and relates more particularly to an improved implantable prosthesis for such splicing.

It is known that the ends of torn off or severed nerves can be made to grow together if a separate growth room, or if one so wishes a scaffold or climbing frame, for the proliferating nerve fibre ends is provided which isolates the nerve portion in question from the other lacerated tissues, such as skin, tendons, blood vessels, etc., which usually appear in connection with the injury causing the nerve rupture.

Thus, in animal experiments gaps of up to about 10 mm between the nerve ends have been made to grow together by fixing the ends in an otherwise empty tube of silicone. By filling the silicone tube with a porous material composed of connective tissue fibres (collagen) from cow skin and glucosaminoglucan from shark cartilage and which is degraded biologically in the body, gaps of about 15 mm have been made to heal within a period of about 6 weeks.

Further, for example, US-A-3,620,218 suggests the use of tubular articles of bioresorbable material, and more specifically of polyglycolic acid (PGA), for nerve splicing. An advantage of such a resorbable tube is, of course, that no foreign material will remain in the body which, at least in the long run, may exert an irritating effect.

The use of bioresorbable polymers for nerve splicing is also disclosed in WO 84/03035. The tubular device shown in this publication is, however, intended for holding nerve ends together end against end without the use of sutures.

A nerve trunk, however, comprises a plurality of nerve fibres, or axons, of different kinds, and it has been found that when using empty or filled prosthetic tubes of the above described types approximately only half, and at best up to 80%, of the nerve fibres in one fracture area will reach the other.

One object of the present invention is to improve the growing together of the nerve fibres in nerve splicing, such that a considerably greater part of the nerve fibres will reach the opposite nerve end.

Another object of the invention to enable bridging of larger distances between the nerve ends than has hitherto been possible.

These objects are achieved by means of a splicing prosthesis which has the features given in the subsequent claims and which is further described hereinafter.

According to a basic concept of the invention the nerve splicing prosthesis, which in its entirety consists of bioresorbable material, comprises an essentially elongate element provided with a plurality of longitudinal channels each intended to receive and form separate growth zones for individual or groups of proliferating nerve fibres.

These longitudinal channels (preferably more than two) may have any cross-sectional configuration, e.g. circular, and are preferably, but not necessarily, completely separated from each other. They need also not be straight but may have a curvilinear extension between the ends of the prosthesis.

Thus, in one embodiment the nerve splicing prosthesis may consist of an elongate cylindrical element provided with a large number of parallel through-channels or bores.

The cross-sectional dimensions of the passages should, of course, be sufficient to permit the passage of the nerve fibres, and they must therefore have a least cross-sectional dimension of about 1 μm. Thus, in the case of, e.g., circular channels a suitable diameter may be from about 5 to about 150 μm, particularly about 5 to about 20 μm.

As is readily appreciated, with regard to the packed arrangement in which the nerve fibres exist in the nerve trunks, the above mentioned channels of the splicing prosthesis should be relatively closely spaced, which means a relatively high perforation degree of the cross-section of the prosthetic element. In the ideal case each nerve fibre should have its own channel, which, however, would hardly be possible to accomplish in practice.

By the term "bioresorbable material" is herein to be understood substantially water-insoluble, non-toxic materials without adverse tissue reaction and which may be degraded and resorbed completely in the body without giving rise to scar tissue or toxic degradation products. Preferably, however, the bioresorbable material is other than proteins, polypeptides and derivatives thereof.

Suitable bioresorbable materials for the purposes of the present invention may easily be selected by the person skilled in the art, for example, among those materials which either are commercially available or have been described in the literature or will be available in the future. Particularly useful are aliphatic polyesters. As examples of bioresorbable materials may be mentioned polymers based upon polyglycolic acid (PGA), copolymers of glycolic acid and lactic acid, copolymers of lactic acid and ε-aminocaproic acid, and various lactide polymerso PGA esters are, e.g., described in the U.S. patent 3,463,658. Copolymers of glycolic acid and lactic acid are, for example, described in the U.S. patent 3,982,543, and homo- and copolymers of lactic acid are described, e.g., in the U.S. patent 3,636,956. Examples of commercially available materials are Vicryl® (a copolymer of 90% glycolic acid and 10% lactic acid, marketed by Ethicon, Sommerville, N.Y., USA - also known as Polyglactin 910) and Dexon® (Davies & Geck, Pearl River, N.Y., USA). Further examples are polydesoxazon (PDS) (Ethicon, USA), polyhydroxybutyric acid (PHB), copolymers of hydroxybutyric acid and hydroxyvaleric acid, polyesters of succinic acid, and cross-linked hyaluronic acid. Of course, also mixtures of the above mentioned materials may be used, for example a mixture of PHB and Vicryl®. The person skilled in the art would easily be able to modify such bioresorbable materials according to the needs in question with regard to the parameters discussed below, such as porosity, resorption period, etc.

The bioresorbable material is preferably porous, particularly microporous, to permit the penetration of fluids.

The strength of the prosthetic material need only be sufficient to withstand the usually relatively low tension load of the spliced nerve.

The resorption period of the prosthetic material should, of course, be sufficient for the proliferation of the nerve fibres, and thus the splicing of the nerve, to be completed before the prosthesis has been resorbed to any substantial extent. This time will, of course, depend inter alia on the distance that the nerve fibres are to grow in the individual case, i.e. the distance between the nerve ends, but the resorption period should usually be longer than about two months.

Optionally one or more growth promoting agents may be incorporated in the prosthetic material, such as nerve growth factor (NGF).

A nerve splicing prosthesis according to the invention may be produced in various ways. For example, it may be produced by packing a number of thin fibres of bioresorbable material into a bundle and then optionally stretching it longitudinally to obtain the desired spacing or passage dimensions between the fibres. The fibres used may be possibly hollow fibres. Such a fibre assembly may optionally be kept together by a resorbable outer covering.

Alternatively, the nerve splicing prosthesis may be produced by drilling with a laser in a solid prosthetic element of a bioresorbable material to make the necessary channels, if necessary followed by longitudinal stretching of the prosthetic element.

When implanting the nerve splicing prosthesis the ends thereof may be attached to the nerve stumps in question in any suitable manner, e.g., by stitching or gluing with a suitable resorbable suture and resorbable glue, respectively.

Preferably the spicing prosthesis is provided in the form of large lengths, optionally in a roll if permitted by the flexibility thereof, from which a suitable prosthesis length is cut at the time of use.

Hereinafter the invention will be described in more detail with regard to a particular embodiment thereof with reference to the accompanying drawings, wherein

Fig. 1 is a perspective view of an embodiment of a nerve splicing prosthesis according to the invention, and
Fig. 2 is a partially transparent schematic perspective view of the nerve splicing prosthesis of Fig. 1 applied to a severed nerve.

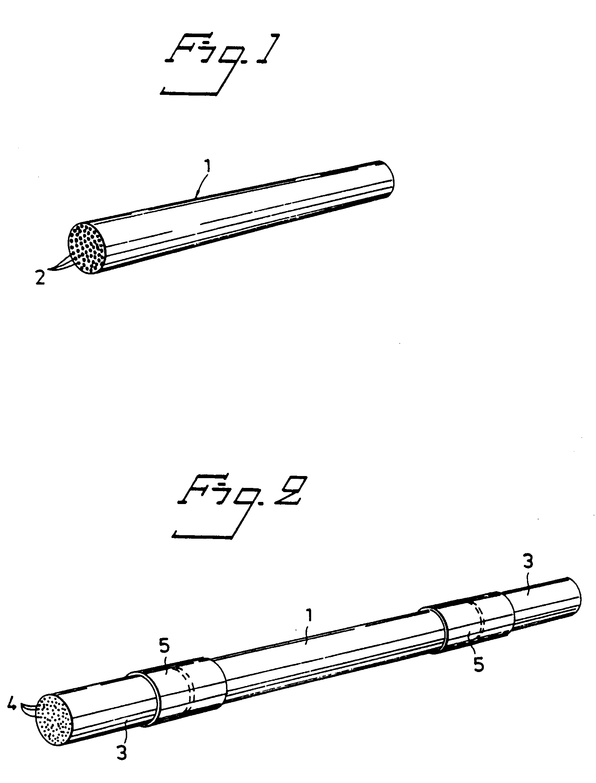

The nerve splicing prosthesis shown in Fig. 1, generally designated by the reference numeral 1, consists of a cylinder of a suitable bioresorbable material, e.g. a microporous material of 85% PHB and 15% Vicryl®, which is provided with a great number of parallel separated channels or bores 2, which for the sake of clarity are shown considerably enlarged and therefore relatively few in number. A suitable diameter of the bores may be about 5-20 μm.

While it is advantageous for the splicing prosthetic element 1 to have substantially the same cross-sectional dimension as the nerve to be spliced, it may be considerably thicker than the nerve without impeding the purposes of the invention to any substantial degree. As an example of a suitable diameter of the nerve splicing prosthesis, about 1 to about 20 mm may be mentioned.

The nerve splicing prosthesis 1 is suitably provided in large lengths, from which a necessary length for the particular application is cut at the time of use.

With reference to Fig. 2, in use a splicing prosthesis 1 of a suitable length is applied to a severed nerve, the two stumps of which are designated by the reference numeral 3 and which has a plurality of nerve fibres or axons schematically indicated by 4. Then, in the illustrated case, the prosthesis 1 is attached to the two nerve ends 3 by means of two fastening sleeves 5 of bioresorbable material, e.g., the same material as that of the prosthesis 1. In this case each fastening sleeve 5 is brought over a nerve end 3 and the adjacent end of the prosthesis 1, whereupon it is fixed to the two parts in any suitable manner, e.g., by stitching with resorbable suture or gluing with a resorbable glue in a manner known per se.

In position the channels 2 of the prosthetic element 1 will serve as defined growth chambers or zones for individual nerve fibres 4 or nerve fibre clusters or bundles. Simultaneously the prosthetic material is slowly resorbed in the body, the resorption period, however, being sufficiently long for the growing together of the nerve to be substantially completed before the intended function of the prosthesis is lost by the resorption. Usually, however, the resorption period would be at least about 2 months.

Below an animal experiment is described in which a nerve splicing prosthesis according to the invention was applied to a sheep.

Example

A nerve splicing prosthesis according to the invention was made from an about 4 mm thick knitted of Vicryl® fibres (56 deniers), onto which PHB had been precipitated to a total wall thickness of about 0.3 mm. The interior of the tube was filled with a bundle of closely packed thin fibres of Vicryl® (about 12,000), which between them defined a very large number of fine channels in the longitudinal direction of the tube.

A two year old sheep was anaesthetized with penthotal and intubated, whereupon the animal was coupled to a respirator for controlled breathing. The narcosis was maintained with oxygen/laughing gas-/fluothane. Nervus ischiadicus was exposed on the back of the thigh, and three centimeters of the nerve were recessed. The nerve splicing prosthesis produced as described above was cut out and fitted into the defect without being tight and was fixed with single resorbable sutures in the outer covering of the prosthesis and the perineurium of the nerve. Finally two fastening sleeves or "muffs", also from knitted Vicryl® fibres with deposited PHB, were slided over the nerve splices and were also fixed with sutures (alternatively fibrin glue). The wound was closed and the animal was waken up, after which it was transferred to a recovery box. After 24 hours the animal was returned to a usual box for normal care with normal diet. Eight weeks after the operation the animal was killed, and the operated region was excised for analysis.

A histological analysis showed good regeneration of axons arranged in bundles in normal manner. The outer polymer layer still remained but was in a state of resolution. No inflammatory reaction was observed.

The invention is, of course, not restricted to the embodiment described above and particularly shown, but many modifications and changes may be made within the scope of the general inventive concept as stated in the claims.

## Claims

1. A prosthesis for splicing nerve ends, comprising an elongate prosthetic element (1) of bioresorbable material, characterized in that it has a plurality of channels (2) which extend between the ends of the prosthetic element (1) and are intended to serve as growth rooms for one or more individual nerve fibres (4) or bundles thereof.

2. A prosthesis according to claim 1, characterized in that said channels (2) are substantially completely separated from each other.

3. A prosthesis according to claim 1 or 2, characterized in that said channels (2) are substantially straight and parallel with the longitudinal axis of the prosthesis.

4. A prosthesis according to any one of claims 1 to 3, characterized in that said channels (2) have a cross-sectional dimension of about 5 to about 150 $\mu$m, preferably about 5 to about 20 $\mu$m.

5. A prosthesis according to any one of claims 1 to 4, characterized in that it has a resorption period of at least about 2 months.

6. A prosthesis according to any one of claims 1 to 5, characterized in that it comprises two fastening sleeves (5) of bioresorbable material for the fixing of the prosthesis (1) to the nerve ends (3).

7. A prosthesis according to any one of claims 1 to 6, characterized in that the prosthetic material that defines said channels (2) is microporous.

8. A prosthesis according to any one of claims 1 to 7, characterized in that said bioresorbable material is other than proteins, polypeptides or derivatives thereof.

9. A prosthesis according to any one of claims 1 to 8, characterized in that said bioresorbable material comprises an aliphatic polyester.

10. A prosthesis according to any one of claims 1 to 8, characterized in that said bioresorbable material is selected from polymers based upon polyglycolic ester, copolymers of glycolic acid and lactic acid, copolymers of lactic acid and ε-aminocaproic acid, homopolymers of lactic acid, lactide polymers, polydesoxazon, polyhydroxybutyric acid, copolymers of hydroxybutyric acid and hydroxyvaleric acid, polyesters of succinic acid, and cross-linked hyaluronic acid.

11. A prosthesis according to any one of claims 1 to 10 in the form of a material length intended to be cut to the desired prosthesis length at the time of use.

## Patentansprüche

1. Prothese zur Verbidung von Nervenenden, mit einem langgestreckten Prothesenelement (1) aus bioresorbierbarem Material, dadurch **gekennzeichnet,** daß sie eine Vielzahl von Kanälen (2) aufweist, die zwischen den Enden des Prothesenelements (1) verlaufen und als Wachstumsräume für eine oder mehrere einzelne Nervenfasem (4) oder Bündel hiervon dienen sollen.

2. Prothese nach Anspruch 1, dadurch **gekennzeichnet,** daß die Kanäle (2) im wesentlichen vollständig voneinander getrennt sind.

3. Prothese nach Anspruch 1 oder 2, dadurch **gekennzeichnet,** daß die Kanäle (2) im wesentlichen geradlinig und parallel zur Längsachse der Prothese verlaufen.

4. Prothese nach einem der Ansprüche 1 bis 3, dadurch **gekennzeichnet,** daß die Kanäle (2) eine Querschnittsabmessung von etwa 5 bis 150 $\mu$m, vorzugsweise etwa 5 bis etwa 20 $\mu$m aufweisen.

5. Prothese nach einem der Ansprüche 1 bis 4, dadurch **gekennzeichnet,** daß sie eine Resorptionszeit von wenigstens zwei Monaten aufweist.

6. Prothese nach einem der Ansprüche 1 bis 5, dadurch **gekennzeichnet,** daß sie zwei Befestigungshülsen (5) aus bioresorbierbarem Material zur Befestigung der Prothese (1) an den Nervenenden (3) aufweist.

7. Prothese nach einem der Ansprüche 1 bis 6, dadurch **gekennzeichnet,** daß das Prothesenmaterial, das die Kanäle (2) bildet, mikroporös ist.

8. Prothese nach einem der Ansprüche 1 bis 7, dadurch **gekennzeichnet**, daß das bioresorbierbare Material ein anderes Material als Proteine, Polypeptide oder deren Derivate ist.

9. Prothese nach einem der Ansprüche 1 bis 8, dadurch **gekennzeichnet**, daß das bioresorbierbare Material einen aliphatischen Polyester enthält

10. Prothese nach einem der Ansprüche 1 bis 8, dadurch **gekennzeichnet**, daß das bioresorbierbare Material ausgewählt ist aus Polymeren auf der Basis von Polyglykolester, Copolymeren von Glykolsäure und Milchsäure, Copolymeren von Milchsäure und ε-Aminocapronsäure, Homopolymeren von Milchsäure, Laktidpolymeren, Polydesoxazon, Polyhydroxybuttersäure, Copolymeren von Hydroxybuttersäure und Hydroxyvaleriansäure, Bernsteinsäurepolyestern und vernetzter Hyaluronsäure.

11. Prothese nach einem der Ansprüche 1 bis 10 in Form eines Materialstranges, der bei Gebrauch auf die benötigte Prothesenlänge ablängbar ist.

## Revendications

1. Prothèse pour raccorder des extrémités de nerfs, comprenant un élément prothétique allongé (1) formé d'un matériau biorésorbable, caractérisée en ce qu'elle possède une pluralité de canaux (2) qui s'étendent entre les extrémités de l'élément prothétique (1) et sont destinés à servir de chambres de croissance pour une ou plusieurs fibres nerveuses individuelles (4) ou des faisceaux de telles fibres.

2. Prothèse selon la revendication 1, caractérisée en ce que lesdits canaux (2) sont sensiblement complètement séparés les uns des autres.

3. Prothèse selon la revendication 1 ou 2, caractérisée en ce que lesdits canaux (2) sont sensiblement rectilignes et parallèles à l'axe longitudinal de la prothèse.

4. Prothèse selon l'une quelconque des revendications 1 à 3, caractérisée en ce que lesdits canaux (2) possèdent une dimension en coupe transversale comprise entre environ 5 et environ 150 $\mu m$ et de préférence entre environ 5 et environ 20 $\mu m$.

5. Prothèse selon l'une quelconque des revendications 1 à 4, caractérisée en ce qu'elle possède une période de résorption égale au moins à environ 2 mois.

6. Prothèse selon l'une quelconque des revendications 1 à 5, caractérisée en ce qu'elle comporte deux manchons de fixation (5) réalisés en un matériau biorésorbable pour la fixation de la prothèse (1) sur les extrémités (3) des nerfs.

7. Prothèse selon l'une quelconque des revendications 1 à 6, caractérisée en ce que le matériau de la prothèse, qui définit lesdits canaux (2), est microporeux.

8. Prothèse selon l'une quelconque des revendications 1 à 7, caractérisée en ce que ledit matériau biorésorbable est formé par un matériau autre que des protéines, des polypeptides ou des dérivés de ces substances.

9. Prothèse selon l'une quelconque des revendications 1 à 8, caractérisée en ce que ledit matériau biorésorbable comprend un polyester aliphatique.

10. Prothèse selon l'une quelconque des revendications 1 à 8, caractérisée en ce que ledit matériau biorésorbable est choisi parmi des polymères à base d'ester polyglycolique, des copolymères de l'acide glycolique et de l'acide lactique, des copolymères de l'acide lactique et de l'acide ε-aminocaproïque, des homopolymères de l'acide lactique, des polymères de lactide, la polydésoxazone, l'acide polyhydroxybutyrique, des copolymères de l'acide hydroxybutyrique et de l'acide hydroxyvalérique, des polyesters de l'acide succinique et l'acide hyaluronique réticulé.

11. Prothèse selon l'une quelconque des revendications 1 à 10, sous la forme d'une longueur de matériau destinée à être coupée à la longueur de prothèse désirée, au moment de l'utilisation.

Fig. 1

Fig. 2